# EUROPEAN PATENT SPECIFICATION

(11) **EP 0 363 211 B1**
(45) Date of publication and mention of the grant of the patent: **06.07.1994**
(21) Application number: 89310233.5
(22) Date of filing: 05.10.1989
(51) Int. Cl.: C07C 401/00, G01N 33/82, G01N 33/60

(54) **1Alpha or 24R,25-Dihydroxy vitamin D3 derivatives, process for their production and assay method using the same**
1-Alpha oder 24R, 25-Dihydroxyvitamin-D3-Derivate, Verfahren zu ihrer Herstellung und Bestimmungsmethode unter Verwendung derselben
Dérives de l-alpha ou 24R, 25-dihydroxy vitamine D3, procédé de leur production et méthode d'essai l'utilisant

(30) Priority: 05.10.1988 JP 251661/88; 11.10.1988 JP 255705/88
(43) Date of publication of application: 11.04.1990
(73) Proprietor: ASAHI KASEI KOGYO KABUSHIKI KAISHA, Osaka (JP)
(72) Inventor: Tanabe, Miyuki, Tagata-gun Shizuoka (JP); Ikuta, Shigeru, Tagata-gun Shizuoka (JP)
(74) Representative: Woods, Geoffrey Corlett

(56) References cited:
- CHEMICAL ABSTRACTS, vol. 111, no. 1, July 3, 1989, Columbus, Ohio, USA; R. RAY et al, "Synthesis and biologic evaluation of a second generation photoaffinity analog of 1,25-dihydroxy-vitamin D3", page 739, abstract-no. 7 667s
- CHEMICAL ABSTRACTS, vol. 106, no. 9, March 2, 1987, Columbus, Ohio, USA; A. KUTNER et al, "Photoactivable analogs for labeling 25-hydroxyvitamin D3 serum binding protein and for 1,25-di-hydroxyvitamin D3 intestinal receptor protein", page 640, abstract-no. 67 565p
- CHEMICAL ABSTRACTS, vol. 105, no. 11, September 15, 1986, Columbus, Ohio, USA; R. RAY et al, "Photoaffinity labeling of the rat plasma vitamin D binding protein with [26,27-3H]-25-hydroxyvitamin D3-3 beta-[N-(4-azido-2-nitro-phenyl)glycinate]", pages 316-7, abstract-no. 93 949r
- CHEMICAL ABSTRACTS, vol. 104, no. 19, May 12, 1986, Columbus, Ohio, USA; R. RAY et al, "Chemical synthesis and biological evaluation of photoaffinity - labeled derivatives of vitamin D3, 25-hydroxyvitamin D3, and 1,25-dihydroxyvitamin D3", page 323, abstract-no. 164 681q
- CHEMICAL ABSTRACTS, vol. 104, no. 3, January 20, 1986, Columbus, Ohio, USA; R. RAY et al, "Evaluation of a photolabile derivative of 1,25-dihydroxyvitamin D3 as a photoaffinity probe for 1,25-dihydroxyvitamin-D3 receptor in chick intestinal cytosol", page 275, abstract-no. 17 260y
- CHEMICAL ABSTRACTS, vol. 103, no. 19, November 11, 1985, Columbus, Ohio, USA; R. RAY et al, "Synthesis of a photoaffinity-labeled analog of 1,25-dihydroxyvitamin D3", page 685, abstract-no. 160 275s

## Description

The present invention relates to 1a or 24R,25-dihydroxy vitamin D₃ derivatives, a process for their preparation and assay methods using then.

Known assay methods of 1a,25-dihydroxy vitamin D₃ include a radio receptor assay (RRA) method and a radio immunoassay (RIA) method. In these methods, a tritium [³H] - labelled compound is used. It is known to use the receptor from a chicken intestine mucosal cytoplasmic fraction in the RRA method [Arch. Biochem. Biophys., 176, 235-243 (1976), Vitamin, 55(12), 595-605: 1981 and Clinical Chem., 1 (1) : 7-18 (1982)]. A compound having a side chain with a carbonyl terminal group is known as a hapten for the preparation of antibodies used in the RIA method [JP-A-58-193463, JP-A-58-92656, JP-A-55-47653, JP-A-61-48497 and JP-A-59-148775, Clinical Science and Molecular Medicine, 54, 329-332 (1978); Clinical Chem., 27/3, 458-463 (1981); Clinical Chem., 26/5, 562-567 (1980); Clinical Chem., 29/1, 196-200 (1983); J. Biochem., 98, 991-998 (1985); and Biochem. Biophys. Res., Comm., 431-436 (1983)]. In these assay methods, tritium [³H] labelled 1a,25-dihydroxy vitamin D₃ is used.

Known assay methods of 24R,25-dihydroxy vitamin D₃ include a competitive protein binding assay (CPBA) method, a tritium [³H]-labelled compound is used. For the vitamin D binding protein (DBP) used in te CPBA method, it is also known to use the plasma of rats which have been fed with vitamin D-deficient feed [Vitamin, 55 (12), 595-605; 1981, Pharma Medica Vol. 1(1): 89-96 (1985) and Hormone and Clinical Trials Vol. 33, No.10: 915-924 (1985)].

In the assay of 1a,25-dihydroxy vitamin D₃ in a specimen, known tritium [³H] - labelled compounds have low specific activity as compared with ³²P or ¹²⁵1 labelled compounds in terms of radiation energy, and suffer from high cost and involve clumbersome procedures with a Liquid Scintillation counter. In the assay of 24R,25-dihydroxy vitamin D₃ in a specimen, the HPLC-UV method is less precise and so the CPBA method has been used. However the tritium [³H]-labelled compound has the same problems as above.

Known tritium labelled vitamin D₃ derivative have only a low rate of β-ray emission. There have been no reports on ¹²⁵1- or ³²P-labelled 1a or 24R,25-dihydroxy vitamin D₃ derivative, which emit y-rays with high energy, since the conjugated triene structure of vitamin D₃ is known to be unstable and thought to be subject to auto-degradation by a radical reaction.

We have discovered an iodine radioisotope-labelled 1a or 24R,25-dihydroxy vitamin D₃ derivative with high radiation energy which has superior characteristics.

The present invention provides an iodine radioisotope-labelled 1a or 24R,25-dihydroxy vitamin D₃ derivative of formula (III): 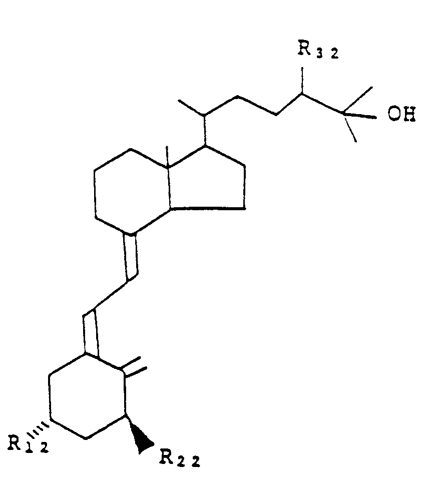 wherein:
R₁₂ is OH or-0-CO-A-NH-Rs;
R₂₂ is H, OH or -O-CO-A-NH-Rₛ; and
R₃₂ is H, OH or -O-CO-A-NH-Rₛ;

wherein A is C₁₋₁₀ alkylene, and R₅ is a group containing an iodine radioisotope, with the proviso that:
1) one of R₂₂ and R₃₂ is H and the other is OH or -O-CO-A-NH-Rs;
2) one of R₁₂ and R₂₂ is OH and the other is -O-CO-A-NH-Rs; or
3) one of R₁₂ and R₃₂ is OH and the other is -O-CO-A-NH-R₅.

The iodine radioisotope is preferably ¹²⁵I. The vitamin D₃ derivative of formula (III) is stable and not subject to structural degradation by y-rays; hence it is useful for use in radio immunoassay techniques.

In radioiosotope labelling techniques, direct labelling (chloramin-T method) and indirect labelling (Bolton-Hunter reagent method) can be used to provide the radioisotope labelled compound (Amersham Note, 1 Nov., 1981).

The indirect labelling method with mild reaction conditions is preferred for preparing the derivatives of the present invention due to the instability of vitamin D against acids, oxygen, oxidizing agents, heat and light.

The derivatives of formula (III) may be prepared from a 1a or 24R,25-dihydroxy vitamin D₃ amino acid derivative having a side chain terminated with an amino group.

The present invention therefore provides an 1 a or 24R,25-dihydroxy vitamin D₃ amino acid derivative of formula (I): 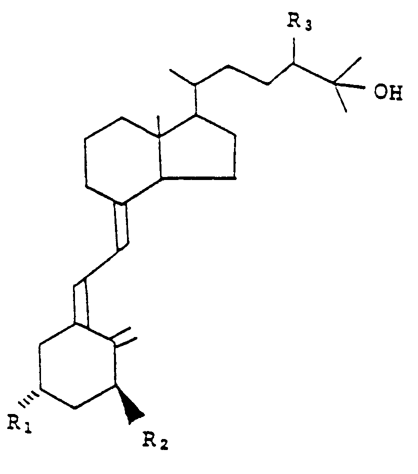 wherein:
R₁ is OH or -0-CO-A-NH₂;
R₂ is H, OH or -0-CO-A-NH₂; and
R₃ is H, OH or -0-CO-A-NH₂;

wherein A is C₁₋₁₀ alkylene, with the proviso that:
1) one of R₂ and R₃ is H and the other is OH or -0-CO-A-NH₂;
2) one of R₁ and R₂ is OH and the other is -0-CO-A-NH₂; or
3) one of R₁ and R₃ is OH and the other is -0-CO-A-NH₂.

The derivative of formula (I) may act as a hapten when it is bound to a carrier protein and inoculated into animals to obtain antibodies. Using the antibodies and the iodine radioisotope-labelled compound, we have also provided a highly sensitive and useful radio immunoassay system of 1a or 24R,25-dihydroxy vitamin D₃ [hereinafter designated as 1a or 24R,25-(OH)₂ D_{3]} in a specimen.

The iodine radioisotope-labelled compound of formula (III) can also be used in a CPBA method using DBP (vitamin D binding p[rotein) for assaying 1a or 24R,25-dihydroxy vitamin D₃ with high sensitivity.

The present invention also provides an assay method for 1a or 24R,25-dihydroxy vitamin D₃ in a specimen, which comprises:
adding to a specimen containing 1a or 24R,25-dihydroxy vitamin D₃ to be assayed, an iodine radioisotopelabelled 1a or 24R,25-dihydroxy vitamin D₃ derivative of formula (III) as defined above;
adding to the mixture thus formed anti-1a or 24R,25-dihydroxy vitamin D₃ antibodies or a vitamin D binding protein to bind competitively to the 1a or 24R,25-dihydroxy vitamin D₃ and to the iodine radioisotope-labelled 1a or 24R,25-dihydroxy vitamin D₃ derivative;
separating the thus produced iodine radioisotope labelled 1a or 24R,25-dihydroxy vitamin D₃ derivative-anti 1a or 24R,25-dihydroxy vitamin D₃ antibody complex or iodine radioisotope-labelled 1a or 24R,25-dihydroxy vitamin D₃ derivative-DBP complex from the iodine radioisotope labelled 1a or 24R,25-dihydroxy vitamin D₃ derivative; and
measuring the amount of iodine radioisotope label in bound or free form.

The 1a or 24R,25-dihydroxy vitamin D₃ amino acid derivative of formula [I] can be obtained by reacting an amino acid with the la-hydroxy or 3β-hydroxy group of 1a,25-dihydroxy vitamin D₃, or with the 24R-hydroxy or 3β-hydroxy group of 24R,25-dihydroxy vitamin D₃. The compound of formula [I] can be used as a hapten in the preparation of anti-1a or 24R,25-dihydroxy vitamin D₃ antibodies. Linkage of the hapten to the carrier protein is preferably by the C₁₋₁₀ alkylene chain.

The compound of formula [I] can be prepared by reacting 1a or 24R,25-dihydroxy vitamin D₃ with an amino acid of formula 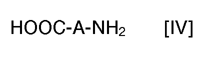 wherein A is as defined above, the amino acid having a protected amino group, to obtain a 1a or 24R,25-dihydroxy vitamin D₃ derivative of formula [II], and removing the protecting group.

Examples of the number of carbon atoms in the A group, and compounds from which they may be obtained, are: n = 1; glycine, n = 2; β-alanine, n = 3; -y-amino-n-butyric acid, n = 4; 6-amino valeric acid, n = 5; _{E}-amino-n-caproic acid, n = 6; 7-amino heptanoic acid, and n = 10; 11-amino undecanoic acid. The carbon chain in A is preferably straight and also preferably contains from 2 to 6 carbon atoms. 6-amino- levulinic acid, glycylglycine or 6-amino acid, i.e. D and/or L-lysine can also be used. An amino acid having a side chain hydroxy group, for example 4-amino-3-hydroxybutyric acid, can be used if the hydroxy group is protected. Thus the alkylene group represented by A may be unsubstituted or substituted, for example by at least one amino, hydroxy or oxo group.

Suitable amino protecting groups are those which can easily be removed in mild conditions such as weakly basic conditions due to the possible instability of vitamin D under more severe removal conditions. Examples are 9-fluorenyl methyloxycarbonyl (hereinafter designated as Fmoc), 9-(2-sulfo)-fluorenyl methyloxycarbonyl, 1,1-dimethyl-2-cyano-ethyloxycarbonyl and 5-benzisoxazolyl methyloxycarbonyl groups, most preferably a 9-fluorenyl methyloxycarbonyl group. An amino protected compound can be made by known methods by reacting an activated derivative, for example an activated ester of an Fmoc containing compound such as N-(9-fluorenylmethyloxycarbonyloxy)-succinimide or 9-fluorenylmethylchloroformate derivative, with an amino acid ([L.A. Carpino and G.Y. Ham, J. Org, Chem. 37: 3404 (1972)].

The 1a or 24R,25-dihydroxy vitamin D₃ amino acid derivative of formula [II] can be obtained, for example, using an Fmoc-amino acid as an amino protected amino acid, by reacting, in the case of 1a,25-dihydroxy vitamin D₃ for covalent bonding via the la-hydroxy or 3β-hydroxy group, and in the case of 24R, 25-dihydroxy vitamin D₃ for covalent bonding via the 24R-hydroxy or 3β-hydroxy group, one equivalent of 1a or 24R,25-dihydroxy vitamin D₃ with one equivalent of Fmoc-amino acid or its acid anhydride, acid halide or activated ester. More preferably, one equivalent of Fmoc-amino acid is reacted with one equivalent of a mixed anhydride with another acid, under an inert gas, and in the presence of a base in an anhydrous solvent. The reason for reacting the mixed anhydride with 1a(or 24R), 25-dihydroxy vitamin D₃ is to prevent the formation of a by-product, 1a (or 24R), 25-dihydroxy vitamin D₃ having an Fmoc-acid group attached to the hydroxy group at position-25.

Examples of other acids in a mixed anhydride are valeric acid, pivalic acid and isobutyloxy carboxylic acid. Pivalic acid is preferred. Examples of anhydrous solvents are anhydrous organic solvents such as tetrahydrofuran or dioxane. Examples of bases are dimethylaminopyridine (DMAP) and piperidinopyridine (PPY), which are preferred for the esterification of secondary or tertiary alcohols with steric hindrance. The preferred inert gases are argon and nitrogen. The reaction preferably proceeds at 0 - 20 °C for 1 - 3 hours. The thus obtained 1a (or 24R), 25-dihydroxy vitamin D₃ derivative of formula [II] is obtained in a mixture with a compound covalently bonding the -O-OC-A-NH-R₄ group [V] , wherein A and R₄ have the same meanings as hereinabove, at the 1a or 3β-position of 1a, 25-dihydroxy vitamin D₃ or 24R -or 3β - position of 24R, 25-dihydroxy vitamin D₃, and can be purified, if necessary, by any conventional purification method such as HPLC.

The 1a (or, 24R), 25-dihydroxy vitamin D₃ of formula [II] is subjected to de-protection of the amino group, i.e. removal of Fmoc, in the presence of a base in an inert solvent to produce a 1a (24R), 25-dihydroxy vitamin D₃ amino acid derivative of formula [I] . Examples of bases are piperidine, morpholine and ethanolamine. Morpholine is preferred. Examples of inert solvents are ethanol and methanol, preferably anhydrous ethanol or anhydrous methanol. The reaction proceeds under an inert gas in the dark at 0 - 20 ° C for 1 - 3 hours.

In the case that the 1a (or 24R), 25-dihydroxy vitamin D₃ derivative of formula [II] is a mixture of compounds in which a residue of formula [V] is covalently bound with the 1a-and 3β-position of 1a, 25-dihydroxy vitamin D₃ or the 24R and 3β-position of 24R, 25-dihydroxy vitamin D₃, the corresponding product of formula [I] can be isolated and purified from the resulting mixture by HPLC. In the case that the derivative of formula [II] is a previously isolated and purified compound, the obtained compound can be purified by column chromatography or TLC.

The by-product which is a compound covalently bonding the residue of formula [V] at the 1a- and 3β-position of 1a, 25-dihydroxy vitamin D₃ or at the 24R-and 3β-position of 24R, 25-dihydroxy vitamin D₃, can be altered to a starting material, 1a (or 24R), 25-dihydroxy vitamin D₃, by hydrolysis with diluted aqueous alkali. A compound of formula [VI]: 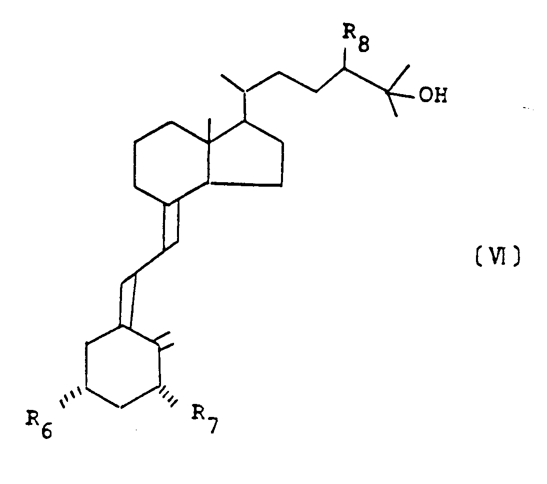 wherein R₆ is -O-CO-A-NH₂, R₇ is hydrogen or R₆, R₈ is hydrogen or R₆, any one of R₇ and R₈ is hydrogen and another is R₆, and A has the same meaning as hereinbefore, can be obtained by treating the by-product with morpholine, and is used for a hapten compound.

In an iodine radioisotope labelled 1a (or 24R), 25-dihydroxy vitamin D₃ derivative of formula [III], A has the same meaning as in formula [I] and R₅ is a group containing an iodine radioisotope. Examples of iodine radioisotopes are ¹²⁵I and ¹³¹I. The relatively long-half-life isotope ¹²⁵I is preferred. Examples of such groups are ₃-(₄-hydroxy-3 -iodo [¹²⁵1] phenyl)-propionyl, 3-(3, 5-diiodo [¹²⁵1] -4-hydroxyphenyl) propionyl, 2-(4-hydroxy-3-iodo [¹²⁵I] plenyl) acetyl, 2-(3, 5-diiodo [¹²⁵I] -4-hydroxyphenyl) acetyl, 2-iodo [¹²⁵I] acetyl, 4-iodo [¹²⁵1] benzoxymethyl carbonyl and an N-substituted-3-iodo [¹²⁵1] tyrosin residue. Among these, 3-(4-hydroxy-3-iodo [¹²⁵I] phenyl) propionyl and 3-(3, 5-diiodo [¹²⁵I] -4-hydroxyphenyl) propionyl are preferred.

In the production of an iodine radioisotope labelled 1a (or 24R), 25-dihydroxy vitamin D₃ derivative of formula [III] , a 1a (or 24R), 25-dihydroxy vitamin D₃ amino acid derivative of formula [I] is labelled with an iodine radioisotope by an indirect labelling method by means of a Bolton-Hunter reagent to obtain the iodine radioisotope labelled 1a (or 24R), 25-dihydroxy vitamin D₃ derivative of formula [III] . An indirect labelling method is a method for producing an iodine radioisotope labelled 1a (or 24R), 25-dihydroxy vitamin D₃ derivative of formula [III] by reacting a reactive derivative of formula [VII] as defined below, which has an iodine radioisotope labelled group, with a 1a (or 24R), 25-dihydroxy vitamin D₃ amino acid derivative of formula [I] .

The reactive derivative of formula [VII] has the formula: 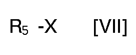 wherein R₅ is as defined above, and X is a succinimidyl-N-oxy, phthalimidyl-N-oxy, 5-norbornene-2, 3-dicarboximidyl-N-oxy or maleimidyl-N-oxy group. N-succinimidyl-3-(4-hydroxy-3-iodo [¹²⁵I] phenyl) propionate, for which R₅ is 3-(4-hydroxy-3-iodo [¹²⁵I] phenyl) propionyl and X is succinylimidyl-N-oxy in the compound of formula [VII] , is a commercially available [¹²⁵I] Bolton-Hunter reagent. An indirect labelling method can be carried out on a 1a (or 24R), 25-dihydroxy vitamin D₃ amino acid derivative of formula [I] using said Bolton-Hunter reagent can be conducted by reacting several p moles to several m mole of radioisotope iodine [¹²⁵1] Bolton-Hunter reagent with a 500 - 2000-fold excess amount, preferably a 1000- fold excess amount, of 1a (or 24R), 25-dihydroxy vitamin D₃ amino acid derivative, at 0 - 30 °C for 12 - 72 hours. In order to increase the effect on the RIA or CPBA method, the produced radioisotope-labelled 1a (or 24R), 25-dihydroxy vitamin D₃ derivative of formula [III] is preferably purified by TLC or HPLC. The thus-obtained ¹²⁵I-labelled 1a (or 24R), 25-dihydroxy vitamin D₃ derivative is stable at -20 ° C in ethanol for more than the two month half-life of 125 and can be used in radio immunoassayes.

The storage temperature is preferably as low as possible, e.g. 5 _{°} C - -20 _{°} C, but preferably even below -20 _{°} C. The derivative may be stored in alcohol or ether, under an inert gas.

Anti 1a (or 24R), 25-dihydroxy vitamin D₃ antibodies can be prepared by inoculating a conjugate of a hapten, i.e. a 1a (or 24R), 25-dihydroxy vitamin D₃ amino acid derivative of formula [I] and a carrier protein into an animal. Examples of carrier proteins which are essential for obtaining an immunogenic antigen for a hapten, are simple proteins, polypeptides and complex proteins such as glycoproteins. Examples of simple proteins are bovine serum albumin (BSA), human serum albumin or human serum globulin. An example of a polypeptide is polylysine. An example of a glycoprotein is mucoprotein. Among these, simple proteins are preferred, and bovine serum albumin and human serum albumin are the most preferred.

The 1a (or 24R), 25-dihydroxy vitamin D₃ amino acid derivative of formula [I] and a carrier protein are covalently bonded in the presence of a condensation reagent or crosslinking reagent. Examples of condensation reagents or crosslinking reagents are dicyclohexylcarbodiimide (DCC), acid anhydride and glutaraldehyde. DCC is preferred. The conjugation ratio of the 1a (or 24R), 25-dihydroxy vitamin D₃ amino acid derivative to the carrier protein can be, due to a decreasing titer of the antibodies if in excess, such that 10 - 40 molecules of 1a (or 24R), 25-dihydroxy vitamin D₃ amino acid derivarive are present per one molecule of carrier protein.

The thus-prepared conjugate for antibody production is inoculated into an animal to produce antibodies. Inoculation can be by parenteral administration such as subcutaneous or intracutaneous injection. A conjugated antigen of the above 1a (or 24R), 25-dihydroxy vitamin D₃ amino acid derivative - carrier protein is dissolved in a buffer solution or physiological saline, together with an equal amount of complete Freund's adjuvant (C.F.A.). The mixture is emulsified completely, and inoculated subcutaneously or intracutaneously into a homeothermal animal, about ten times in every 1 - 3 weeks to immunize the same. Alternatively, the conjugated antigen can be directly inoculated into a spleen. During the immunization period, the serum antibody titer is measured at constant time intervals; at maximum titer, a whole blood sample is collected and allowed to stand for coagulation. The coagulated sample is centrifugally separated to obtain an antiserum containing anti - 1a (or 24R), 25-dihydroxy vitamin D₃ antibodies.

The type of the homeothermal animal is not limited; it can be any animal which has antibody production activity. To obtain large amounts of antibodies, a goat, sheep or bovine animal is preferably used. In general, a chicken, rabbit, rat or mouse is used.

The isolation of anti -1a (or 24R), 25-dihydroxy vitamin D₃ antibody from the antiserum can be performed by any conventional method for antibody purification. For example, ammonium sulfate fractionated antiserum can be treated by ion-exchange chromatography or gel-filtration.

Another method of the production of the antibodies is by innoculating spleen cells, which can produce the desired antibodies of the animal, with the conjugated antigen of 1a (or 24R), 25-dihydroxy vitamin D₃ amino acid derivative - carrier protein, and fusing the cells with established myeloma cells. The thus-obtained hybridoma is cultured and the monoclonal antibodies produced by the hybridoma are used.

For example, an emulsion, which is prepared by mixing a conjugate antigen of 1a (or 24R), 25-dihydroxy vitamin D₃ amino acid derivative - carrier protein dissolved in buffer solution or physiological saline and an equal amount of C.F.A., is inoculated subcutaneously in mice, for example of strain Balb/c for sensitization with several times inoculation every week. Cell-fusion is performed 3 - 5 days after final sensitization. At 3 - 5 days after final sensitization, spleen cells which produce anti - 1a (24R), 25-dihydroxy vitamin D₃ antibodies are collected and fused with established myeloma cells which can be cultured for a long term. Long-term culturable established cells may be defined as cells which can be cultured and grown for a long term in vitro or in vivo, and which can produce immunoglobulin or its related proteins. Generally, well-grown myeloma cells are used. Preferred myeloma cells are cell-lines P3-NSI/1-Ag4-1, P3-X63-Ag8U1, SP2/U-Ag14 and MPC11-45.6.TG.1.7. In the present invention, P3-X63-Ag8U1 is preferred. The cells can be cultured in a conventional cell culture medium. For example culture can be performed in a medium of 10% FCS to which is added RPMI 1640 (tradename, Flow Laboratory) to which is added added glutamine, pyruvic acid, penicillin and streptomycin. For stock culture, S-azaguanine is added to the above medium. 1 - 3 x 10^{s} myeloma cells are used for cell fusion. Spleen cells can be prepared by cutting a mouse spleen and crushing it on a mesh to prepare a spleen cell suspension. Washed cells, generally 1 - 3 x 10^{s} cells, are fused with myeloma cells by mixing together. The ratio of mixed cells can be myeloma cells:spleen cells 1 : 3 - 10. Cell fusion is achieved in a medium for hybridomas. A conventional cell-fusion method using a promoter such as Sendai virus or polyethylene glycol (PEG) is preferred. PEG is particularly preferred.

The fused cells are inoculated into the medium for hybridomas and incubated, then selected by incubating in HAT medium. HAT medium is a medium for hybridomas with added hypoxathine, aminopterin and thymidine. Since more than two hybridomas can be grown in the well of a cell-separation plate, more than two kind of antibodies are possibly produced, or no-antibody producing cells may be a contaminant. In order to obtain cells having the same properties, each clone should be separated. For cloning, a limiting dilution culture or soft agar culture is used. In this invention, a limiting dilution culture is preferred.

The thus-obtained hybridoma secreting anti-1a (or 24R), 25-dihydroxy vitamin D₃ antibodies with high titer can be stored after lyophilization at am early stage. Lyophilization can be performed by a conventional method, namely cell suspension in a small tube or ampule with freezing in a -80 _{°} C freezer and storage in liquid nitrogen. Another example of hybridoma production is that the above hybridoma is inoculated intraperitoneally in pristan (2, 6, 10, 14-tetramethylpentadecane, Aldrich Chemicals) treated mice, and after about 10 days the ascites are collected. Another method is that the hybridoma is incubated into bovine fetal serum with added RPMI medium or into Darbecco-modified Eagle medium. The antibodies thus obtained can be purified by any conventional method. For example, the antibodies are fractionated with ammonium sulfate and treated by ion-exchange chromatography, gelfiltration and affinity chromatography to fractionate IgG. Then purified anti- 1a (or 24R), 25-dihydroxy vitamin D₃ monoclonal antibodies can be obtained.

Furthermore, anti- 1a (or 24R), 25-dihydroxy vitamin D₃ monoclonal antibody-producing cells can be inoculated and grown in an animal having identical histocompatibility antigens or in nude mice as a tumor, the grown cells are collected and the monoclonal antibodies separated therefrom.

In an assay of 1a (or 24R), 25-dihydroxy vitamin D₃, anti- 1a (or 24R), 25-dihydroxy vitamin D₃ polyclonal antiboies or anti- 1a (or 24R), 25-dihydroxy vitamin D₃ monoclonal antibodies (hereinafter sometimes collectively designated anti- 1a (or 24R), 25-dihydroxy vitamin D₃ antibody) can be used in a soluble state or in an immobilized state. An insoluble carrier and anti- 1a (or 24R), 25-dihydroxy vitamin D₃ antibodies are bound using a polyfunctional reagent and the immobilized antibody has an antibody titer against 1a (or 24R), 25-dihydroxy vitamin D₃. Examples of polyfunctional reagents are compounds having more than two functional groups which can react with functional groups such as amino, hydroxyl, carboxyl and thiol, and comprise aldehydes such as succinaldehyde, glutaraldehyde and adipoaldehyde; dicarboxylates such as malonic acid, succinic acid, glutaric acid, or adipic acid or their reactive derivatives; diisocyanates such as hexamethylene diisocyanate or 2, 4-toluenediisocyanate; diisothiocyanates such as hexamethylene diisothiocyanate; maleimide carboxylates such as maleimide benzoate or maleimide phenylacetate or their functional derivatives; dimaleimides such as N, N-ethylene-bis-maleimide or N, N'-O-phenylene dimaleimide, bisdiazobenzidine, diethylmalonimidate, dimethyladipinimidate or N, N'-polymethylene-bisiodo acetamide and thiocarboxylates such as 3-(2'-benzothiozolyl-dithio) propionate and 3-(2'-pyridyldithio) propionate or their functional derivatives. The polyfunctional reagent can be selected by considering the bonding of functional groups such as amino, carboxyl, hydroxyl or thiol in an anti- 1a (or 24R), 25-dihydroxy vitamin D₃ antibody.

The immobilized carrier is a carrier having a reactive group which does not bond with groups for bonding with antibodies in polyfunctional groups. Examples of immobilized carriers are insoluble proteins such as albumin or gelatin; epichlorhydrin-treated insoluble polysaccharides such as agarose, cellulose or dextrin; insoluble polymers or copolymers of acrylonitrile, acrylic acid, aclylate ester, methacrylic acid, methacrylate ester, vinyl alcohol, vinyl acetate, styrene, aminostyrene, chlorstyrene, maleic acid and fumaric acid, which is treated with bromocyanate and is introduced with a spacer corresponding to amino group introduction, and an insoluble inorganic carrier which is introduced with a functional group such as an amino group into an inorganic compound of, for example, silicon or aluminium. The immobilized carrier may also be a carrier which can bind anti-1a (or 24R), 25-dihydroxy vitamin D₃ antibodies by physical adsorption.

The immobilized carrier is preferably in particulate form which can be easily isolated by filtration, for example beads having a diameter of more than 1 mm, preferably more than 5 mm, or in a spindle form which corresponds to the bottom shape of an antigen-antibody reaction tube.

The introduction of a reactive group into anti- 1a (or 24R), 25-dihydroxy vitamin D₃ antibodies using a spacer-introducing reagent can be performed by introducing additional functional groups such as aldehyde, carboxyl, amino or thiol, reacting with at least one spacer introducing reagent, for example a dialdehyde such as succinaldehyde, glutaraldehyde or adipoaldehyde; a reactive derivative such as acid chloride, a succinimide ester or p-nitrophenyl ester of m-amino butyric acid or m-amino glutamic acid; a reactive derivative of dicarboxylic acids such as malonic acid, succinic acid, glutaric acid or adipic acid; diamines such as hexamethylene diamine or decamethylene diamine; reactive derivatives of 3-(2'-pyridyl-dithio) propionic acid or 3-(2'-benzothiazolyldithio) propionic acid, S-acetyl-mercapto succinic anhydride or thiols such as 2-aminoethanethiol.

Anti - 1a (or 24R), 25-dihydroxy vitamin D₃ antibodiesare condensed directly or through polyfunctional reagents with reactive groups in the immobilized carrier. The condensation generally proceeds at 0 - 40 _{°} C in a pH 6.0 - 8.5 buffer solution or organic solvent or mixture thereof. Furthermore, a second antibody which is obtained by immunizing large mammals inoculated with an immunoglobulin fraction in serum, which is used for antibody production of 1a (or 24R), 25-dihydroxy vitamin D₃, is immobilized and anti - 1a a (or 24R), 25-dihydroxy vitamin D₃ antibody is bound thereto by an antigen-antibody reaction to prepare the immobilized antibody.

The thus-obtained immobilized antibodies are washed and stored.

In an assay of 1a (or 24R), 25-dihydroxy vitamin D₃ in a specimen using anti -1a (or 24R), 25-dihydroxy vitamin D₃ antibodies in a liquid soluble phase, a fixed amount of iodine radioisotope labelled 1a (or 24R), 25-dihydroxy vitamin D₃ derivative of formula [III] is first added to a specimen containing 1a (or 24R), 25-dihydroxy vitamin D₃, then an optimum amount of anti- 1a (or 24R), 25-dihydroxy vitamin D₃ antibodies is added to form an antigen antibodies reaction product.

The thus-formed labelled 1a (or 24R), 25-dihydroxy vitamin D₃ derivative-anti-1a (or 24R), 25-dihydroxy vitamin D₃ antibodies binding complex, the 1a (or 24R), 25-dihydroxy vitamin D₃-antibodies binding complex, and the free labelled 1a (or 24R), 25-dihydroxy vitamin D₃ derivative are separated using specific antibodies for anti- 1a (or 24R), 25-dihydroxy vitamin D₃ antibodies. Said specific antibodies are hereinafter referred to as second antibodies. The second antibodies can be obtained, for example, by inoculating a normal immunoglobulin fraction in the serum of an animal which is used for antibodies production of 1a (or 24R), 25-dihydroxy vitamin D₃, as an antigen to immunize, then isolating the second antibodies from the thus-obtained antiserum. The second antibodies can be purified if necessary by any known method, or they can preferably be used in the state of an antiserum.

An assay method for 1a (or 24R), 25-dihydroxy vitamin D₃ in a specimen using anti- 1a (or 24R), 25-dihydroxy vitamin D₃ antibodies and iodine radioisotope labelled 1a (or 24R), 25-dihydroxy vitamin D₃ derivative is illustrated as follows:
1a (or 24R), 25-dihydroxy vitamin D₃ in a specimen such as a known serum is extracted from the serum specimen. A sample, which is a mixture of serum and an equal amount of added solvent, is stirred, allowed to stand and centrifuged. The separated supernatant solution is treated by column chromatography and a fraction containing 1a (or 24R), 25-dihydroxy vitamin D₃ is collected and preferably purified by HPLC.

The 1a (or 24R), 25-dihydroxy vitamin D₃ fraction can be checked by previously added tritum [³H] -labelled 1a (or 24R), 25-dihydroxy vitamin D₃. The 1a (or 24R), 25-dihydroxy vitamin D₃ fraction is dried in vacuo, the vacuum is flushed with argon gas and the fraction is dissolved in ethanol to prepare a specimen. A fixed amount of iodine radioisotope labelled 1a (or 24R), 25-dihydroxy vitamin D₃ derivative is added to a specimen, and the most suitable amount of anti-1a (or 24R), 25-dihydroxy vitamin D₃ antibody is added thereto. The mixture is incubated in a medium for antigen-antibodies such as a phosphate buffer or a veronal buffer at 4 - 5_{°}C for about 15 - 72 hours to promote the competitive reaction of iodine radioisotope labelled antigen and non-labelled antigen to antibodies. The thus-formed antigen-antibody binding complex, namely a bound form of iodine radioisotope labelled 1a (or 24R), 25-dihydroxy vitamin D₃ derivative - anti- 1a (or 24R), 25-dihydroxy vitamin D₃ antibody (B) and an unreacted free form of iodine radioisotope labelled 1a (or 24R), 25-dihydroxy vitamin D₃ derivative (F) are separated by the dextran- charcoal (DC) method by filtration or by centrifugation at 3,000 r.p.m. for 15 mins. Upon B-F separation, the radioactivity of each of B and F is measured.

The amount of 1a (or 24R), 25-dihydroxy vitamin D₃ (H) in a specimen is calculated by measuring the radioactivity and calculating B/(B + F) or B/F. When the amount of H is increased, the radioactivity of B is decreased and that of F is increased. Therefore, the unknown amount H can be determined by measuring the radioactivity of B and F from a previously plotted standard curve of known amounts.

Upon B-F separation, when the double antibody technique is used with soluble state antibodies, second antibodies, preferably second antibidies containing an antiserum IgG and if necessary a normal serum IgG of the same kind of animal used for anti- 1a (or 24R), 25-dihydroxy vitamin D₃ antibodies production, are added after the competitive reaction and incubated for 1 - 12 hours. Thereafter the formed binding complex is precipitated by centrifugation at 3,000 r.p.m. for 10 - 30 minutes to separate precipitate (B) and supernatant (F), and then the radioactivity of B or F is measured.

According to the present assay method, a standard curve for 2 pg/Test - 256 pg /Test can be prepared, and a rapid reaction time of 16 hours at 5 _{°} C or 1 hour at 37 _{°} C can be achieved. Furthermore, the operation after the reaction is quite simple. Moreover, dilution and recovery tests using the specimens display linearity with high precision.

When the antibodies are replaced by known DBP, it can be reacted with an iodine [¹²⁵1] radioisotope labelled 1α (or 24R), 25-dihydroxy vitamin D₃ derivative and a standard curve for the range 1 - 32 pg/Test can be prepared. The DBP can be obtained from the serum of chickens, rats, mice, rabbits, goats, sheep, bovine animals or humans.

The following Examples further illustrate the present invention.

### Referential Example 1:

### Extraction and purification of 1a, 25-dihydroxy vitamin D₃ from serum:

A mixture of serum (0.5mℓ) and acetonitrile (0.5 mℓ) was stirred in a BORTEX mixer and then allowed to stand for 30 minutes. The supernatant solution (0.8 m1) obtained by centrifugation at 3,000 r.p.m. for 10 mins. was charged on a Sept-pack C-18 cartridge column (tradename, MILLIPORE, Waters Corp.) which was activated with ethanol, equilibrated with 50% acetonitrile, and eluted with hydrated acetonitrile. The column was then washed with 50 % acetonitrile (4 m1) then eluted with 64% acetonitrile (4 m1) (a fraction of 1a, 25-dihydroxy vitamin D₃), and eluted with 73% acetonitrile (4 mt)(a fraction of 25-hydroxy vitamin D₃ and 24R, 25-dihydroxy vitamin D₃). The fraction eluted with 64% acetonitrile was dried in vacuo and flushed with argon gas to obtain a fraction containing 1a, 25-dihydroxy vitamin D₃. The thus-obtained crude fraction was dissolved in a mixture (200 µℓ) of n-hexane-isopropanol (9 : 1) and purified by HPLC in a Zorbax-SIL (Dupont Inc.) 0.46 x 25 cm column. The above fractions of 1a, 25-dihydroxy vitamin D₃ were checked by previously added tritium [³H] -labelled 1a, 25-dihydroxy vitamin D₃ [26, 27-methyl-³H]. The fraction of 1a, 25-dihydroxy vitamin D₃ (Rt = 9 - 13 sins.) was dried in vacuo and flushed with argon gas.

The said fractions were dissolved in ethanol and 10µ ℓ thereof was used for an assay.

The recovery of 1a, 25-dihydroxy vitamin D₃ was 95 ± 2 % (n = 10).

### Referential Example 2:

### 3-(N-fluorenyl methyloxy carbonyl) amino propionic acid:

y-amino-propionic acid (β-alanine, 178 mg, 2 m mole) was added to N-(9-fluorenylmethyloxycar- bonyloxy)-succinimide (674 mg, 2 m mole) dissolved in a mixture of tetrahydrofuran (THF)-dimethylformamide (DMF)-H₂0 (1:2:2) (25 m1) and the mixture was reacted at room temperature overnight. The reaction solvent was distilled off in vacuo and the residue was charged on a column of silica-gel (Wako-gel C-200 , 75 g) and separated and purified by eluting with CHCℓ₃: methanol = 9 : 1 to obtain the above compound (538.8 mg) (yield 86.5 %).
NMR. 8 (DMSO-d6) ppm
2. 50-2. 60 (2H, t, -CH₂-CO-)
3. 28-3. 35 (2H, m, -CH₂-N-)
4. 33-4. 44 (3H, m, Fmoc)
7. 35-8. 06 (8H ,m, Fmoc)

### Example 1:

### 1a, 25-dihydroxy vitamin D₃- 3β- [3 -(N-fluorenylmethyloxy carbonyl) aminopropionate ] [hereinafter designated as 1a, 25-(OH)₂ D₃-3β-O-Ala-Fmoc ] and 1a, 25-dihydroxy vitamin D₃- 1a - [3-(N-fluorenylmethyloxycarbonyl) aminopropionate ] [hereinafter designated as 1a, 25-(OH)₂ D₃ -1a- O- Ala-Fmoc ]:

Pivaloyl chloride (Trimethyl acetylchloride 3.07 µℓ, 0.025 m mole) and dimethylamino pyridine (DMAP, 3.05 mg, 0.025 m mole) were added to 3-(N-fluorenyl methyloxy carbonyl) amino propionic acid (Fmoc-β-Ala] (7.775 mg, 0.025 m mole) dissolved in dry THF (2 mt), and the mixture was reacted at-15°C for 15 mins. 1a, 25-dihydroxy vitamin D₃ (10.4 mg, 0.025 m mole) was added thereto and the mixture was reacted at -15_{°}C for 5 mins. and at 0°C for 1.5 hours. Methanol (0.5 mℓ) was added to the mixture, then distilled off in vacuo. The residue was separated by silica-gel column chromatography [Wako gel C-200, 150 g, developer: ethylacetate-n-hexane (4 : 1) ] to obtain the three fractions hereinbelow:
Fraction 1:
   1a, 25-(OH)₂ D₃-bis (β-Ala-Fmoc) [= 1a, 25-dihydroxy vitamin D₃- 1a, 3β-bis [3-(N-fluorenyl methyloxy carbonyl) amino] propionate,
   Yield : 19.1 % (Rf : 0.59 )
Fraction 2:
   A mixture of 1a, 25-(OH)₂ D₃ -3β-O- β-Ala-Fmoc and 1a, 25- (OH)₂ D₃ - 1a-0 - β-Ala-Fmoc,
   Yield : 20.3 % (Rf : 0.47 )
Fraction 3 :
   Starting material, 1a, 25-(OH)₂ D₃,
   Yield : 41.2 % (Rf : 0.20)
   Fraction 2 was treated with HPLC (Zorbax-ODS; φ 0.46 x 25 cm, developer: 10 % H₂0-methanol; elution: 1 mℓ/ mins.) to separate each component.

The physical properties of each product are shown hereinbelow:
1a, 25-(OH)₂ D₃- 3 β-O - β-Ala-Fmoc:
HPLC Rt = 17. 46 mins.
NMR. δ (CDCℓ₃) ppm
0. 55 (3H, s), 0. 94 (3H, d), 1. 22 (6H, s), 2. 53 (2H, t), 3. 44 - 3. 48 (3H, m), 4. 22 (1 H, t), 4. 37 - 4. 41 (3H, m), 5. 03 (1 H, d), 5. 27 - 5. 30 (2H, m), 5. 35 (1 H, m), 6. 01 (1 H, d), 6. 34 (1 H, d), 7. 29 - 7. 77 (8H, m) 
EtOH
U V λ m a x
 (nm) 300. 5, 266. 3 214. 6 1a, 25-(OH)₂ D₃ - 1a-0 -β-Ala-FmOC:
HPLC Rt = 14. 92 mins.
NMR. δ (CDCℓ₃) ppm
0. 50 (3H, s), 0. 90 (3H, d), 1. 20 (3H, s), 1. 27 (3H, s), 4. 04 - 4. 05 (1 H, m ), 4. 21 (1 H, m ), 4. 41 - 4. 46 (2H, m), 5. 05 (1 H, d), 5. 27 (1 H, t), 5. 32 (1 H, m), 5. 50 (1 H, t), 5. 90 (1 H, d), 6. 30 (1 H, d), 7. 28 - 7. 79 (8H, m) 
EtOH
U V m a x
 (nm ) 300. 4, 266. 4, 214. 3
1a, 25-(OH)₂ D₃ -bis (β-Ala-Fmoc) :
NMR. δ (CDCℓ₃) ppm
0. 50 (3H, s), 0. 90 (3H, d), 1. 20 (3H, s), 1. 26 (3H, s), 2. 40 - 2. 65 (4H, m), 3. 30 - 3. 55 (4H, m), 4. 10 - 4. 50 (6H, m), 5. 05 - 5. 60 (6H, m), 5. 90 (1 H, d), 6. 32 (1 H, d), 7. 20 - 7. 83 (16H, m)

### Example 2 :

### (1) 1a, 25-dihydroxy vitamin D₃- 3β-O-(3-aminopropionate) [hereinafter designated as 1a, 25-(OH)₂ D₃- 3β-O-β-Ala] :

1a, 25-(OH)₂ D₃- 3β-Ala-Fmoc (13.4 mg, 18.86 x 10-³ m mole) was dissolved in ethanol (2.5 mt). Morpholine (2.5 mt) was added thereto and the mixture was stirred at room temperature for 2 hours, under an argon atmosphere. The reaction mixture was concentrated in vaccuo to obtain a crude product and purified by LH-20 (H₂0, 10 mℓ, trade name) and washed with water, 20 - 30 mℓ to remove morpholine. The product was eluted with methanol and the thus eluted product was charged on Amprep-NH₂ -(Amarsham Corp. 500 mg, n-hexane-dichloromethane 1 : 1) which was washed with n-hexane-dichloromethane (1 : 1), 30 mt, then eluted with ethanol to obtain the compound.

Yield : 8.73 x 10 -³ m mole (Yield : 46.25 %) 
EtOH
U V  λ max  :
 264. 2 nm

Ninhydrin colorization : positive

TLC (Silica-gel, Merck 5715, developer: ethylacetate-methanol = 1 : 4) Rf : 0.15

### (2) 1a, 25-dihydroxy vitamin D₃-1 a-o-3-aminopropionate [hereinafter designated as 1a, 25- (OH)₂ D₃ -1a-o-β-Ala] :

In the above (1), 1a, 25-(OH)₂ D₃ -3β-O -β-Ala-Fmoc was replaced by 1a, 25-(OH)₂ D₃ - 1a-0 - β-Ala-Fmoc to obtain the desired product.

Yield : 62.5 % 
EtOH
U V  λ max :
 265. 4nm

Ninhydrin colorization : positive

TLC (Silica-gel, Merk 5715, ethylacetate-methanol = 1 : 4): Rf : 0.25

### Example 3 :

### 1a, 25-dihydroxy vitamin D₃ - 3β - [3-(BSA-amino) propionate] antigen and 1a, 25-dihydroxy vitamin D₃ antiserum:

### (1) Preparation of 1a, 25-dihydroxy vitamin D₃ -3β-[3-(BSA-amino) propionate] antigen.

1a, 25-(OH)₂ D₃ - 3β- O- β - Ala (12.2 mg, 25.05 x 10 -³ m mole) obtained by removal of Fmoc was dissolved in THF (1 mt), and was added to BSA (M.W. 65,000, 32.6 mg, 1 / 50 x 25.05 x 10 -³ m mole) dissolved in tris buffer (0.1 M, pH 8.6, 10 mt) at 0°C with 1-ethyl-3-(3-dimethylaminopropyl) carbodiimide hydrochloride (6.3 mg, 1.3 x 25.05 x 10 -³ m mole). The reaction mixture was reacted at 0 °C for 3 hours. Chloroform (10 mt) was added four times to the reaction mixture and unreacted 1a, 25-(OH)₂ D₃ - 3β-O -β-Ala was removed. The aqueous layer was freeze dried to obtain the lyophilized product (28.0 mg) which was a complex of 18 molecules of 1a, 25-(OH)₂ D₃ -3β-O -β-Ala bound via an amino group at position-3 thereof to one molecule of BSA.

### (2) Preparation of 1a, 25-dihydroxy vitamin D₃ antiserum:

The lyophilized 1a, 25-dihydroxy vitamin D₃ -3β-[3-(BSA-amino) propionate] (antigen) obtained in (1) above was dissolved in tris buffer (0.1 M, pH 8.6). An equal amount of C.F.A. was added thereto and the material was mixed to emulsify it so as to prepare antigen 1 µg - 100 µg/mℓ. The emulsion was inoculated subcutaneously ten times each two weeks, 10 y - 200 y/head, into rabbits. During this immunization, the liter of blood samples collected every ten days was measured and at maximum antibody liter the whole blood was collected. The blood samples were allowed to stand at room temperature for 60 mins. to coagurate and were centrifuged at 3,000 r.p.m. for 10 mins. to obtain an antiserum containing anti- 1a, 25-dihydroxy vitamin D₃ antibodies, which was fractionated with ammonium sulfate to collect an IgG fraction.

### (3) Preparation of anti- 1a, 25-dihydroxy vitamin D₃ monoclonal antibodies :

Lyophilized 1a, 25-dihydroxy vitamin D₃- 3β - [3 -(BSA-amino) propionate ] (antigen) obtained in (1) above dissolved in phosphate buffer, pH 7.2, (50 y ) together with C.F.A. was inoculated subcutaneously into Balb/c mice, female, 4 weeks old. After 1 week, 50 y thereof was subcutaneously irtoculated and after 2 weeks 50 y of antigen was intraperitoneally inoculated. Three days after the final inoculation, the mice spleens were finely cut and crushed on a mesh to prepare a spleen cell suspension. Cell fusion was performed using mouse myeloma cells P3- X63-Ag8U1 by a conventional method. 30% PEG (M.W. 1,000) aqueous solution was incubated at 37 °C. The above spleen cells and myeloma cells (total 4 x 10⁷ cells, 5:1) were suspended in RPMI medium (5 mℓ) and both cells were gently mixed, centrifuged at 1,000 r.p.m. for 10 mins., then the supernatant was vacuum filtered. The test tube was gently shaken to mix the cell pellets, PEG solution (1.0 mℓ) was slowly added and the mixture was gently stirred. The mixture was incubated at 37°C with gentle shaking, then the fusion reaction was stopped by adding slowly a conventional medium (10 mℓ) and again the cells were suspended. The suspension was centrifuged at 1,000 r.p.m. for 5 mins. The precipitated cell pellet was dispersed by gently shaking and slowly suspended in HAT medium (5 mℓ) and was transferred into HAT medium (1 mℓ) in a vessel. The cells were observed microscopically. The cell suspension was pipetted (each 200 µℓ) into wells in a 96-well plate and grown in a C0₂-incubator. The fused cells were selected in HAT medium for cloning by a limiting dilution technique. The obtained clone suspension was grown in the peritonea of pristan-treated Balb/c mice. The IgG fraction was collected by a conventional method from ascites and serum and purified by affinity chromatography using protein A-bound Sepharose CL-4B. The thus obtained IgG subclass was IgG 1 which was used as monoclonal antibodies.

### Examples 4 :

### (1) Preparation of iodine [¹²⁵1] radioisotope labelled 1a, 25-dihydroxy vitamin D₃ derivative; 1a, 25-dihydroxy vitamin D₃- 3 β- {3 - [N-3-(4-hydroxy-3-iodo [¹²⁵1] phenyl) propionyl ] amino propionate } :

Iodine [¹²⁵1] Bolton-Hunter reagent [NEN, NEX-120-10, 2200 Ci/m mole, 0.33 mCi/100 µℓ benzene: 50 µCi] and THF (75 µℓ) triethylamine (EtₐN, 300 p mole) were added to a ethanol solution of 1a, 25-(OH)₂ D₃ -3β-O -β-Ala (72.8 n mole/25 µℓ EtOH) obtained in example 3, (1) and the mixture was reacted at room temperature for 24 hours under an argon atmosphere. The reaction mixture was purified by HPLC.
Radio-recovery : 27.4 µCi (54.7 %)
HPLC:
   ① Column : Zorbax-ODS; φ 4.6 x 250 mm 5 µm
      Elution: 15% H₂0-methanol, 1 mt/mins.
         Rt : 13.5 - 15.6 mins.
   ② Column : Zorbax-SIL; φ 4.6 x 250 mm 5 µm
      Elution: 20% isopropanol-n-hexane;
         1 mt/mins. ; Rt : 16 - 22 mins.

### (2) Preparation of 1a, 25-dihydroxy vitamin D₃ - 1a-{3- [N-3-(4-hydroxy-3-iodine [¹²⁵1] phenyl) pro- pionylamino] propionate} :

In (1) hereinabove, 1a, 25-(OH)₂ D₃ - 3β-O - β-Ala was replaced by 1a, 25-(OH)₂ D₃ - 1a-0 -β-Ala to obtain the desired product.
Radio recovery : 30.2 µCi (60.4 %)
HPLC :
   ① The same condition as above (1) :
      Rt : 14.2 - 16.8 mins.
   ② The same condition as above (2) :
      Rt : 13.6 - 16.1 mins.

### Example 5:

### (1) Production of a mixture of 1a, 25-(OH)₂ D₃-3β-O-β-Ala and 1a, 25- (OH)₂ -1a-0 -β-Ala:

Morpholine (2 mℓ) was added to a mixture (8.4 mg, x 10 -³ m mole) of 1a, 25-(OH)₂ D₃ -3β-Ala-Fmoc and 1a, 25-(OH)₂ D₃ - 1a-0 -β-Ala-Fmoc, dissolved in ethanol (2 mt), at room temperature and reacted at room temperature for 2 hours under an argon atmosphere. The reaction solvent was removed in vacuo and the residue was purified by LH-20 (H₂0, 10 mℓ) (Pharmacia Corp.) as in Example 3,(1).

The residue was further purified by Amprep-NH₂ (Amersham Corp., 500 mg) as of in Example 3,(1).

Yield : 5.13 x 10 -³ m mole (43.4 %)

[mixture containing approx. 2.5 : 1 of 1a, 25-(OH)₂ D₃ -3β -O -β-Ala and 1a, 25-(OH)₂ D₃-1α-O -β-Ala] 
EtOH
UV λmax :
 265.0 nm

Ninhydrin colorization : positive

TLC (Silica-gel, Merck 5715, ethyl acetate-methanol = 1 : 4) : Rf : 0.15 [ 1a, 25-(OH)₂ D₃ -3β-O - β-Ala], 0.25 [ 1 a, 25-(OH)₂ D₃ -1α-O - β-Ala]

### (2) Preparation of iodine [¹²⁵1] radioisotope labelled 1a, 25-dihydroxy vitamin D₃ -3β - {3- [N-4-hydroxy -3-iodo [1251] phenyl) propionyl ] amino propionate (3β-labelled compound) and 1a, 25-dihydroxy vitamin D₃ -1a- {3- [N-4-hydroxy-3-iodo [¹²⁵1] phenyl) propionyl ] amino propionate (la-labelled compound):

Iodine [¹²⁵1] Bolton-Hunter reagent [NEN, NEX-120-10, 2200 Ci / m mole, 0.33 mCi / 100 µℓ benzene: 100 µCi], THF (30 µℓ) and triethylamine (600 p mole) were added to an ethanol solution (205 n mole/100 µℓ ethanol) of the mixture obtained in above (1), and the mixture was reacted at room temperature for 24 hours under an argon atmosphere. The reaction mixture was purified by HPLC.
Radio recovery : 50.7 µCi (50.7 %)
HPLC :
   ① the same condition as above (1)
      Rt : 13 - 17 mins.
   ② the same condition as above (2)
      Rt : 13 - 22 mins.

3β-labelled compound and 1a-labelled compound were separated and purified by HPLC with the condition ②.
1α-labeled compound: Rt : 13.0 - 15.6 mins. radio recovery : 16.3 µCi
3β-labeled compound: Rt : 16.0 - 21.2 mins. radio recovery : 34.5 µCi

Ratio of recovery of 1a-and 3β -compound: 1 : 2.1 which was identical with the ratio in the starting material (before de-Fmoc)

### Example 6 :

### Preparation of standard curve using radio immunoassay of 1a, 25-dihydroxy vitamin D₃:

A standard ethanol solution of 1a, 25-dihydroxy vitamin D₃ was diluted stepwise by two-fold dilution to prepare 1024 pg/ 20 µℓ , 512 pg/ 20 µℓ , 256 pg/ 20 µℓ , 128 pg/ 20 µℓ , 64 pg/ 20 µℓ , 32 pg/ 20 µℓ, 16 pg/ 20 µℓ, 8 pg/ 20 µℓ, and 4 pg/ 20 µℓ, and O (for Bo) and 2048 pg/ 20 µℓ, (for NSB) samples.

Samples (20 µℓ) of each concentration hereinabove were pipetted into two tubes. Ethanol solutions, each 20µℓ, of iodine [¹²⁵1] radioisotope labelled compound (3β-labelled compound) were pipetted into each tube (20 µℓ, ca. 12,000 cpm) except samples Bo and NSB.

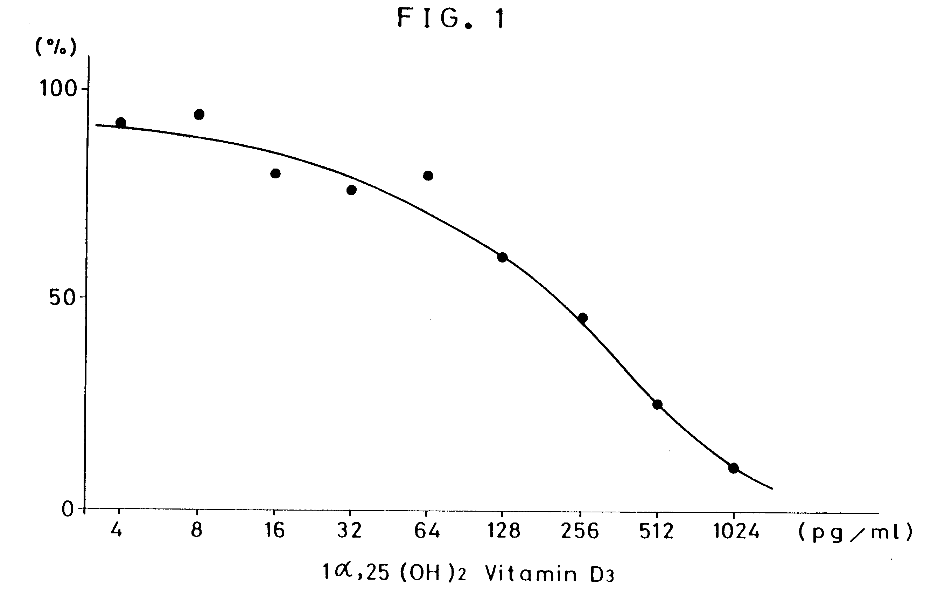

Anti-1a, 25-dihydroxy vitamin D₃ sheep serum diluted with tris buffer pH 8.6, each 1 mℓ, was added to each tube. Each mixture in the tubes was incubated at 5°C overnight for 16 - 24 hours. A mixture of dextran and active charcoal, each 200 µℓ, was added into the reaction tubes. B-F separation was performed by centrifugation at 5 °C for 30 mins., and each supernatant solution (1 mℓ) was measured by autowell y-counter. The binding ratio was calculated by the following equation: 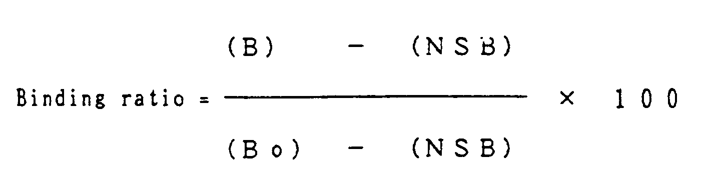 wherein (B) was the count number in each tube, (NSB) was the count number measured using 1a, 25-dihydroxy vitamin D₃ (2048 pg/20 µℓ) in place of ¹²⁵1 labelled 1a, 25-dihydroxy vitamin D₃ derivative, and (Bo) was the count number measured using 1a, 25-dihydroxy vitamin D₃ at zero concentration. Fig. 1 shows the standard curve of [¹²⁵I] - labelled 1a, 25-dihydroxy vitamin D₃ derivative.

### Referential Example 3:

### Extraction and purification of 24R 25-dihydroxy vitamin D₃ in serum:

A mixture of serum (0.5 m) and acetonitrile (0.5 mℓ) was stirred in a BORTEX mixer and then allowed to stand for 30 mins. The supernatant solution (0.8 m1) obtained by centrifugation at 3,000 r.p.m. for 10 mins. was charged on a Sep-pack C-18 cartridge column (tradename, MILLIPORE, Waters Corp.) which was activated with ethanol and equilibrated with 50% acetonitrile, and eluted with hydrated acetonitrile. The column was further washed with 50% acetonitrile (4 m1) then eluted with 64% acetonitrile (4 m1) (a fraction of 1a, 25-dihydroxy vitamin D₃), and eluted with 73% acetonitrile (4 m1) (a fraction of 25-hydroxy vitamin D₃ and 24R, 25-dihydroxy-vitamin D₃). The fraction eluted with 73% acetonitrile was dried in vacuo and flushed with argon gas to obtain a fraction containing 24R, 25-dihydroxy vitamin D₃. The thus-obtained crude fraction was dissolved in a mixture (200 µℓ) of n-hexane-isopropanol (9 : 1) and purified HPLC in a Zorbax-SIL (Dupont Inc.) 0.46 x 25 cm column. The above fraction of 24R, 25-dihydroxy vitamin D₃ was checked by previously added tritium [³H] -labelled 24R, 25-dihydroxy vitamin D₂ [26, 27-methyl-³H]. The fraction of 24R, 25-dihydroxy vitamin D₃ (Rt = 5 - 7 mins.) was dried in vacuo and flushed with argon gas.

The said fractions were dissolved in ethanol (200 mℓ) and 20 µℓ thereof was used for an assay.

The recovery of 24R, 25-dihydroxy vitamin D₃ gas 92% (n = 18).

### Example 7:

24R 25-dihydroxy vitamin D₃ - 3β- [3-(N-fluorenylmethyloxy carbonyl) aminopropionate] (hereinafter designated as 24R, 25-(OH)₂ -D₃- 3β-O -β-Ala-Fmoc ] and 24R, 25-dihydroxy vitamin D₃-24R - [3-(N-fluorenyl methyloxy carbonyl) aminopropionate ] [hereinafter designated as 24R, 25-(OH)₂ D₃ -24R-O -β-Ala-Fmoc ]

Pivaloyl chloride (4.62 µℓ, 1.5 x 0.025 m mole) and dimethylamino pyridine (DMAP, 4.57 mg, 1.5 x 0.025 m mole) were added to 3-(N-fluorenyl methyloxy carbonyl) amino propionic acid (hereinafter designated as β -Ala-Fmoc)] (11. 6 mg, 1.5 x 0.025m mole) dissolved in dry THF (2 mi), and the mixture was reacted at -10°C, under an argon atmosphere to produce mixedunhydride of β-Ala-Fmoc and pivaloyl chloride. The reactionmixture was kept at -10°C for approx. 10 mins. and 24R, 25-dihydroxy vitamin D₃ -(10.4 mg, 0.025 m mole) was added thereto and the mixture was reacted at 0°C for 30 mins. then at room temperature for 1 hour. The reaction mixture was concentrated in vacuo after removing a precipitate, then purified by preparative silica-gel TLC [Merck 57 17, 20 x 20 (H) cm, ethyl acetate-n-hexane = 2 : 1] to obtain the mixture of 24R, 25-(OH)₂ D₃ -3β-O-Ala-Fmoc and 24R, 25-(OH)₂ D₃ -24R-O- β-Ala-Fmoc, a starting material 24R, 25-(OH)₂ D₃ and 24R, 25-dihydroxy vitamin D₃-bis [3-(N-fluorenyl methyloxy carbonyl) amino ] propionate [hereinafter designated as 24R, 25-(OH)₂ D₃ -bis ( β-Ala-Fmoc ].

Yield:
24R, 25-(OH)₂ D₃-3β-O -β-Ala-Fmoc 43.1 %
24R, 25-(OH)₂ D₃-24R-O -β-Ala-Fmoc 12.7 %
24R, 25-(OH)₂ D₃-bis (β-Ala-Fmoc) 33.5%
recovered starting material 24R, 25-(OH)₂ D₃ 10.8 %

Physical properties :
24R, 25-(OH)₂ D₃ -3β-Ala-Fmoc:
NMR δ (CDCℓ₃) ppm
0. 55 (3H, s), 0. 94 (3H, d), 1. 17 (3H, s), 1. 21 (3H, s), 2. 17 - 2. 24 (1 H, m), 2. 35 - 2. 42 (2H, m), 2. 53 - 2. 60 (3H , m), 2. 77 - 2. 81 (1 H, m), 3. 31 - 3. 35 ( 1 H, m), 3. 44 - 3. 49 (2H, m), 4. 19 - 4. 38 (3H, m), 4. 85 (1 H, d), 4. 98 - 5. 02 (1 H, m), 5. 07 (1 H, m), 5. 27 - 5. 30 (1 H, b), 6. 03 (1 H, d), 6. 21 (1 H, d ), 7. 29 - 7. 77 (8H, m) 
EtOH
U V λ m a x
 (nm) 299. 7, 265. 2
HPLC;
   ① Zorbax-SIL 5 % isopropanol-n-hexane, 2 mℓ /mins.
      Rt : 14.0 mins.
   ② Zorbax-ODS 10 % H₂0 (CH₃CN - methanol = 1 : 1),
      2 mℓ /mins. Rt : 8.4 mins.
24R, 25-(OH)₂ D₃ - 24R-O-β-Ala-Fmoc:
NMR δ (CDCℓ₃) ppm
0. 52 (3H, s), 0. 92 (3H, d), 1. 19 (3H, s), 1. 21 (3H, s), 2. 15 - 2. 17 (1 H, m), 2. 25 - 2. 28 (1 H, m), 2. 30 - 2. 38 (1 H , m), 2. 55 - 2. 61 (3H, m), 2. 79 - 2. 83 ( 1 H, m), 3. 48 - 3. 56 (2H, m), 3. 93 - 3. 95 (1 H, m), 4. 20 - 4. 39 (3H, m) , 4. 80 (1 H, d), 4. 82 - 4. 85 (1 H, m), 5. 02 (1 H, m), 5. 49 (1 H, b), 6. 01 (1 H, d), 6. 22 (1 H, d), 7. 29 - 7. 77 (8H, m) 
EtOH
U V λ m a x
 (nm) 299, 6, 265. 3
HPLC;
   ① condition, the same as above (1), Rt : 18.4 mins.
   ② condition, the same as above (2) , Rt : 6.5 mins.
      24R, 25-(OH)₂ D₃ -bis (β-Ala-Fmoc):
      NMR δ (CDCℓ₃) ppm
      0. 52 (3H, s), 0. 92 (3H, d), 1. 19 (3H, s), 1. 21 (3H, s), 2. 17 - 2. 22 (1 H, m), 2. 35 - 2. 41 (2H, m), 2. 53 - 2. 61 (5H, m), 2. 75 - 2. 78 (1 H, m), 3. 44 - 3. 57 (4H, m), 4. 18 - 4. 39 (6H, m), 4. 83 - 4. 85 (2H, m), 4. 98 - 5. 01 (1 H, m), 5. 04 (1 H, m), 5. 28 - 5. 30 (1 H, b), 5. 48 - 5. 51 (1 H, b), 6. 00 (1 H, d), 6. 20 (1 H, d), 7. 29 - 7. 7 7 (16H, m)

### Example 8 :

### (1) 24R, 25-dihydroxy vitamin D₃-3β-(3-aminopropionate) [hereinafter designated as 24R, 25-(OH)₂ D₃-3β-O -β -Ala] :

24R, 25-(OH)₂ D₃- 3β-O -β-Ala-Fmoc (2.4 mg, 3.38 x 10 -³ m mole) obtained in Example 7 was dissolved in ethanol (1 m ℓ). Morpholine (1 m ℓ) was added thereto and the mixture was reacted at room temperature for two hours. The reaction mixture was concentrated in vacuo and purified by LH-20 (H₂0 8 mt, tradename) and washed with purified water (30 mt) to remove morpholine. The product was eluted with methanol and the thus eluted product was concentrated and charged on Amprep-NH₂ (Amersham Corp., 500 mg, n-hexane-dichloromethane 1 : 1) which was washed with n-hexane-dichloromethane (1 : 1), 20 mt, then eluted with ethanol to obtain the compound.

Yield: 25. 4 % 
EtOH
U V λ m a x   :
 264. 6nm

Ninhydrin colorization: positive

### (2) 24R, 25-dihydroxy vitamin D₃ -24R-0-3-aminopropionate [hereinafter designated as 24R, 25-(OH)₂ D₃-24R-O -β-Ala ] :

In the above (1), 24R, 25-(OH)₂ D₃- 3β-O -β-Ala-Fmoc was replaced by 24R, 25-(OH)₂ D₃-24R -0 - β-Ala-Fmoc to obtain the desired product.

Yield: 40 % 
EtOH
U V λ m a x :
 265. 4nm

Ninhydrin colorization: positive

### Example 9 :

### (1) Preparation of iodine [¹²⁵1] radioisotope labelled 24R, 25-dihydroxy vitamin D₃ derivative; 24R, 25-dihydroxy vitamin D₃ - 3β- {3- [N-(4-hydroxy-3-iodo [¹²⁵1] phenyl) propionyl ] amino propionate} :

Iodine [¹²⁵1] Bolton-Hunter reagent (NEN, NEX-120-10, 2200 Ci/m mole ] (50 µCi/30 pM/15 µl benzene), THF (20 µl) and triethylamine 300 p mole) were added to an ethanol solution of 24R, 25-(OH)₂ D₃ - 3β-O -β-Ala (66.35 n mole /100 µl EtOH) obtained in Example 8,(1) and the mixture was reacted at room temperature for 24 hours under an argon atmosphere in the dark. The reaction mixture was purified by HPLC.

HPLC:
① Zorbax-ODS; 15% H₂0-methanol, 1 mt/mins.
   ②Rt : 12.0 - 13.3 mins.
0 Zorbax-SIL; 20% isopropanol-n-hexane, 1 ml/mins.
   Rt : 12.6 - 17.2 mins.

Radio-recovery: 29.9 µCi (59.7 %)

### (2) Preparation of 24R, 25-dihydroxy vitamin D₃-24R-O-[3- {N-3-(4-hydroxy-3-iodine [¹²⁵1] phenyl) pro- pionylamino} propionate] :

In (1) hereinabove, 24R, 25-(OH)₂ D₃ - 3β-O -β -Ala was replaced by 24R, 25-(OH)₂ D₃ - 24R -O -β-Ala obtained in Example 8, (2) to obtain the product.

HPLC:
① Conditions, the same as above ①
   ② Rt : 9.9 - 11.3 mins.
0 Condition, the same as above ②:
   Rt : 11.3 - 14.8 mins.
      Radio-recovery : 53.9 %

### Example 10 :

### Preparation of standard curve of 24R, 25-(OH)₂ D₃ using iodine [¹²⁵1] radioistope labelled 24R, 25-(OH)₂ D₃:

A standard ethanol solution of 24R, 25-(OH)₂ D₃ (1.024 pg/20 µl) was diluted stepwise by two-fold dilutions to prepare 128 pg/20 µl, 64 pg/20 µl, 32 pg/20 µl, 16 pg/20 µl, 8 pg/20 µl, 4 pg/20 µl, 2 pg/20 µl, and 0 pg/20 µl.

Samples (20 µl) of each concentration hereinabove were pipetted into two tubes. Aliquots of an ethanol solution, each 20 µl, of iodine [¹²⁵1] radioisotope labelled 24R, 25-(OH)₂ D₃ derivative, containing as an antioxidant tocopherol, were pipetted into each tube (20 µl, ca. 13,000 cpm). DBP diluted with tris buffer, pH 8.6 each 1 ml, stirred and allowed to stand at 5 °C for overnight (20 - 24 hours).

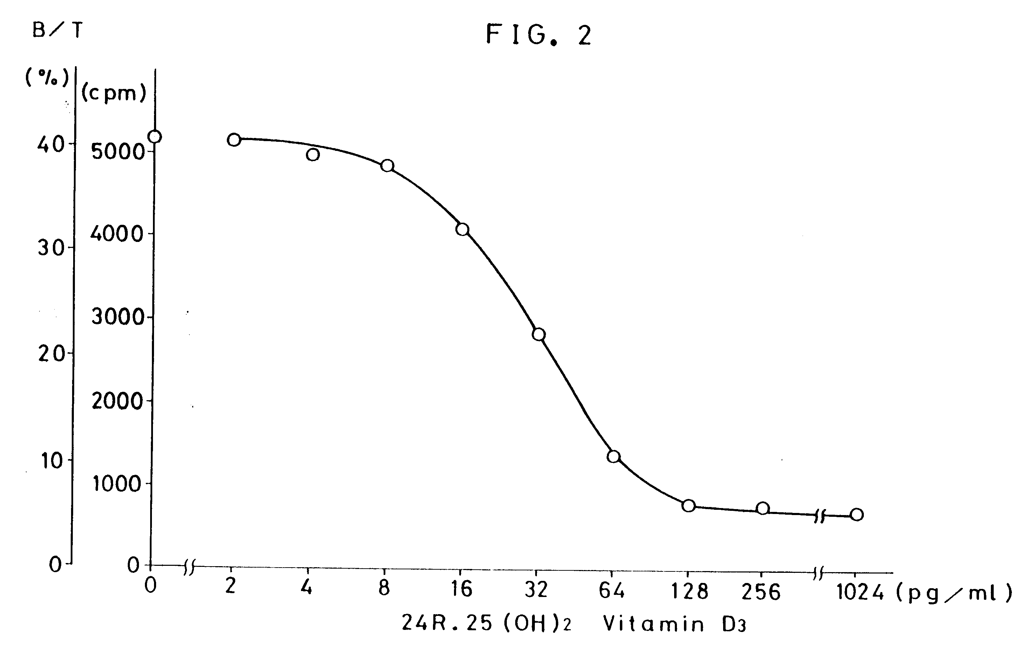

A solution of dextran and active charcoal (200 µl), was added into the reaction tubes, stirred and incubated at 5 °C for 30 mins. with stirring at 15 mins. intervals. B-F separation was made by centrifugation at 3,000 r.p.m. for 15 mins., and each supernatant solution (1 ml) was measured by an Autowell y-counter. The standard curve was prepared by plotting an average count obtained in each tube. The result is shown in Fig. 2.

The present invention provides iodine radioisotope labelled 1a, 25-dihydroxy vitamin D₃ derivatives with high radiation energy and wide usability, compared to low radiation energy, β-ray emitting tritium [³H] -labelled vitamin D₃. It might be thought that high radiation energy radioisotope iodine [¹²⁵1] or phosphorus [^{32p} ] vitamin D₃ would degrade automatically due to a reaction on the conjugated double bond in the vitamin D structure, but the [¹²⁵1] -labelled 1a (or 24R), 25-dihydroxy vitamin D₃ derivative of the present invention in fact does not autodegrade by y-rays and is quite stable for use in a radio immunoassay.

In general, vitamin D₃ is metabolized in vivo in the liver or kidney and is converted to activated vitamin D₃. The activated vitamin D₃, such as 25-hydroxy vitamin D₃, 1a, 23-dihydroxy vitamin D₃, la-hydroxy vitamin D₃ and 1a, 24-dihydroxy vitamin D₃, is used clinically for the therapy of osteoporosis and osteomalacia. The clinical dose thereof is quite low due to its strong physicological action. The pharmacological activity is correlated to the blood level and tissue level of the drug and hence measurement of the blood level of the drug administered to humans is important from a clinical point of view. Considering the above, the present invention is valuable for the assay of activated vitamin D₃ in clinical tests.

In the accompanying drawings :
Figure 1 is the standard curve of 1a, 25-dihydroxy vitamin D₃ using an [¹²⁵1] -labelled 1a, 25-dihydroxy vitamin D₃ derivative ; and
Figure 2 is the standard curve of 24R, 25-dihydroxy vitamin D₃ using an [¹²⁵1] -labelled 24R, 25-dihydroxy vitamin D₃ derivative.

## Claims

1. A 1a or 24R,25-dihydroxy vitamin D₃ amino acid derivative of formula(I): 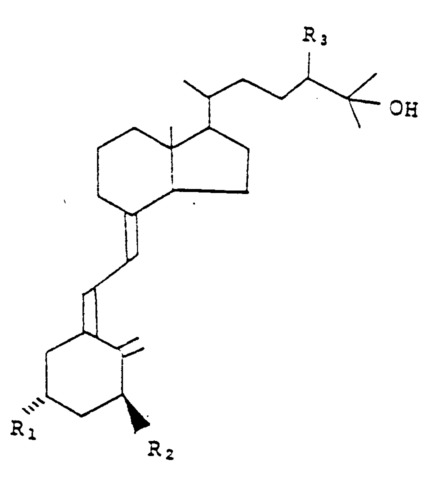 wherein:
R₁ is OH or -O-CO-A-NH₂;
R₂ is H, OH or -0-CO-A-NH₂; and
R₃ is H, OH or -O-CO-A-NH₂;
wherein A is C₁ -₁₀ o alkylene, with the proviso that:
(1) one of R₂ and R₃ is H and the other is OH or -0-CO-A-NH₂;
(2) one of R₁ and R₂ is OH and the other is -0-CO-A-NH₂; or
(3) one of R₁ and R₃ is OH and the other is -0-CO-A-NH₂.

2. A process for the production of vitamin D₃ amino acid derivative of formula (I) as defined in claim 1, which comprises removing, in the presence of a base and in an inert solvent, an amino protecting group from a vitamin D₃ derivative of formula (II): 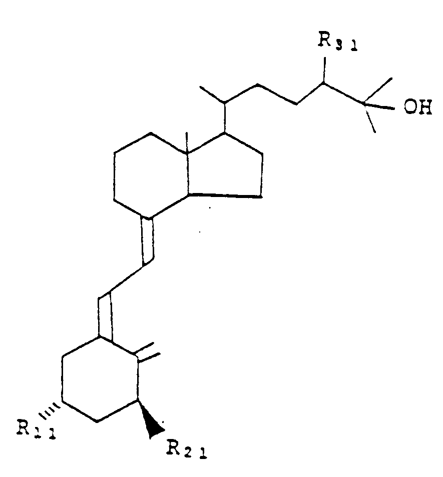 wherein:
R₁₁ is OH or -O-CO-A-NH-R₄;
R₂₁ is H, OH or -O-CO-A-NH-R₄; and
R₃₁ is H, OH or -O-CO-A-NH-R₄;
wherein A is C₁₋₁₀ alkylene and R₄ is an amino protecting group, with the proviso that:
(1) one of R₂₁ and R₃₁ is H and the other is OH or -O-CO-A-NH-R₄;
(2) one of R₁₁ and R₂₁ is OH and the other is -O-CO-A-NH-R₄; or
(3) one of R₁₁ and R₃₁ is OH and the other is -O-CO-A-NH-R₄.

3. A process according to claim 2 wherein R₄ is 9-fluorenylmethyloxycarbonyl.

4. An iodine radioisotope-labelled 1a or 24,25-dihydroxy vitamin D₃ derivative of formula (III): 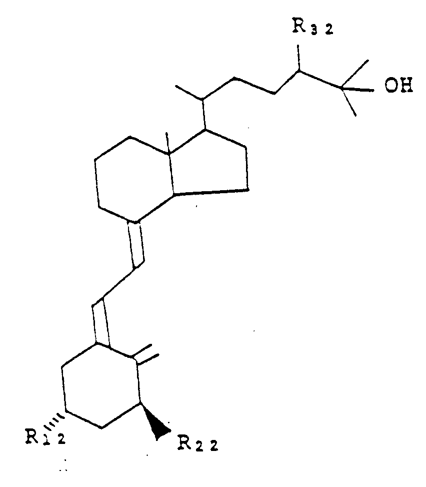 wherein:
R₁₂ is OH or -0-CO-A-NH-R₅;
R₂₂ is H, OH or -0-CO-A-NH-R₅; and
R₃₂ is H, OH or -0-CO-A-NH-R₅;
wherein A is C₁₋₁₀ alkylene and R₅ is a group containing an iodine radioisotope, with the proviso that:
(1) one of R₂₂ and R₃₂ is H and the other is OH or -0-CO-A-NH-R₅;
(2) one of R₁₂ and R₂₂ is OH and the other is -0-CO-A-NH-R₅; or
(3) one of R₁₂ and R₃₂ is OH and the other is -0-CO-A-NH-R₅.

5. A vitamin D₃ derivative according to claim 4 wherein the iodine radioisotope is ¹²⁵ I.

6. A vitamin D₃ derivative according to claim 5 wherein the gorup containing an iodine radioisotope is 3-(4-hydroxy-3-iodo(¹²⁵I)-phenyl)-propionyl or 3-(3,5-diiodo(¹²⁵I)-4-hydroxyphenyl)-propionyl.

7. An assay method for 1a or 24R,25-dihydroxy vitamin D₃ in a specimen which comprises:
adding, to a specimen containing 1a or 24R,25-dihydroxy vitamin D₃ to be assayed, an iodine radioisotope-labelled 1a or 24,25-dihydroxy vitamin D₃ derivative as defined in any one of claims 4 to 6;
adding to the mixture thus formed anti-1a or 24R,25-dihydroxy vitamin D₃ antibodies or a vitamin D binding protein to bind competitively to the 1a or 24,25-dihydroxy vitamin D₃ and to the iodine radioisotope-labelled 1a or 24,25-dihydroxy vitamin D₃ derivative;
separating the thus produced iodine radioisotope-labelled 1a or 24R,25-dihydroxy vitamin D₃ derivative-anti 1a or 24R,25-dihydroxy vitamin D₃ antibody complex or iodine radioisotope-labelled 1a or 24R,25-dihydroxy vitamin D₃ derivative-DBP complex, from the iodine radioisotope-labelled 1a or 24R,25-dihydroxy vitamin D₃ derivative; and
measuring the amount of iodine radioisotope label in bound or free form.

8. A process for preparing an iodine radioisotope-labelled 1α or 24,25-dihydroxy vitamin D₃ derivative as defined in any one of claims 4 to 6, wherein a 1α or 24,25-dihydroxy vitamin D₃ derivative as defined in claim 1 is reacted with a compound of formula (VII):
Rs-X
wherein R₅ is as defined in claim 4 and X is a succinimidyl-N-oxy, phthalimidyl-N-oxy, 5-norbornene-2,3-dicarboximidyl-N-oxy or maleimidyl-N-oxy group.

## Patentansprüche

1. 1a- oder 24R,25-Dihydroxy-Vitamin-D3-Aminosäurederivat der Formel (I): 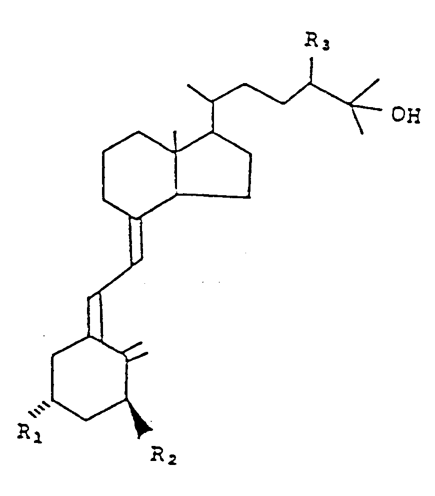 worin:
R₁ OH oder -O-CO-A-NH₂ bedeutet,
R₂ H, OH oder -O-CO-A-NH₂ bedeutet, und
R₃ H, OH oder -O-CO-A-NH₂ bedeutet,
wobei A einen C_{1 -10} -Alkylenrest bedeutet, mit der Maßgabe, daß:
(1) einer der Reste R₂ und R₃ H bedeutet und der andere OH oder -O-CO-A-NH₂ bedeutet,
(2) einer der Reste R₁ und R₂ OH bedeutet und der andere -O-CO-A-NH₂ bedeutet oder
(3) einer der Reste R₁ und R₃ OH bedeutet und der andere -O-CO-A-NH₂ bedeutet.

2. Verfahren zur Herstellung eines Vitamin-D3-Aminosäurederivates mit der in Anspruch 1 definierten Formel (I), das die Entfernung einer Aminoschutzgruppe von einem Vitamin-D3-Derivat der Formel (II): 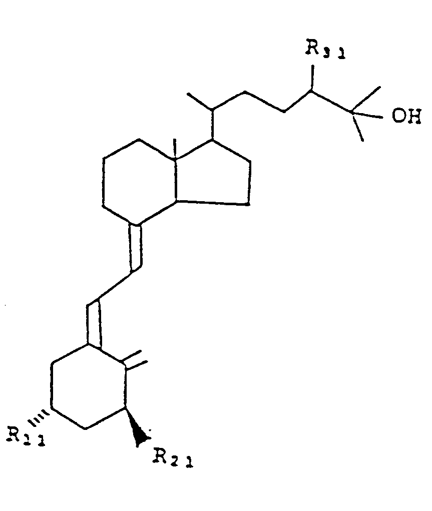 worin:
R₁₁ OH oder -O-CO-A-NH-R₄ bedeutet,
R₂₁ H, OH oder -O-CO-A-NH-R₄ bedeutet, und
R₃₁ H, OH oder -O-CO-A-NH-R₄ bedeutet,
wobei A einen C₁₋₁₀-Alkylenrest bedeutet und R₄ eine Aminoschutzgruppe bedeutet, mit der Maßgabe, daß:
(1) einer der Reste R₂₁ und R₃₁ H bedeutet und der andere OH oder -O-CO-A-NH-R₄ bedeutet,
(2) einer der Reste R₁₁ und R₂₁ OH bedeutet und der andere -O-CO-A-NH-R₄ bedeutet oder
(3) einer der Reste R₁₁ und R₃₁ OH bedeutet und der andere -O-CO-A-NH-R₄ bedeutet, in Gegenwart einer Base und eines inerten Lösungsmittels umfaßt.

3. Verfahren nach Anspruch 2, worin R₄ eine 9-Fluorenylmethyloxycarbonyl-Gruppe bedeutet.

4. lod-Radioisotop-markiertes 1a- oder 24R,25-Dihydroxy-Vitamin-D3-Derivat der Formel (III): 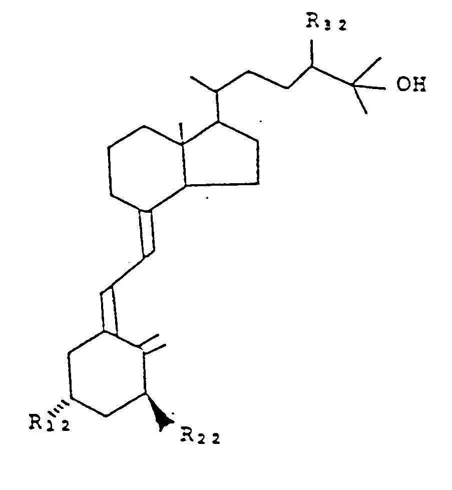 worin:
R₁₂ OH oder -O-CO-A-NH-R₅ bedeutet,
R₂₂ H, OH oder -O-CO-A-NH-R₅ bedeutet, und
R₃₂ H, OH oder -O-CO-A-NH-R₅ bedeutet,
wobei A einen C₁ -, ₒ-Alkylenrest bedeutet und R₅ eine lod-Radioisotop-enthaltende Gruppe bedeutet, mit der Maßgabe, daß:
(1) einer der Reste R₂₂ und R₃₂ H bedeutet und der andere OH oder -O-CO-A-NH-R₅ bedeutet,
(2) einer der Reste R₁₂ und R₂₂ OH bedeutet und der andere -O-CO-A-NH-R₅ bedeutet oder
(3) einer der Reste R₁₂ und R₃₂ OH bedeutet und der andere -O-CO-A-NH-R₅ bedeutet.

5. Vitamin-D3-Derivat nach Anspruch 4, worin das lod-Radioisotop ¹²⁵1 bedeutet.

6. Vitamin-D3-Derivat nach Anspruch 5, worin die lod-Radioisotop-enthaltende Gruppe eine 3-(4-Hydroxy-3-iodo(¹²⁵I)-phenyl)-propionyl- oder eine 3-(3,5-Diiodo(¹²⁵I)-4-hydroxyphenyl)-propionyl-Gruppe bedeutet.

7. Analyseverfahren für 1a- oder 24R,25-Dihydroxy-Vitamin-D3 in einer Probe, umfassend:
die Zugabe eines nach einem der Ansprüche 4 bis 6 definierten lod-Radioisotop-markierten 1a-oder 24R,25-Dihydroxy-Vitamin-D3-Derivats zu einer zu analysierendes 1a- oder 24R,25-Dihydroxy-Vitamin-D3 enthaltenden Probe,
die Zugabe von anti-1a- oder -24R,25-Dihydroxy-Vitamin-D3-Antikörpern oder eines Vitamin-D3 bindenden Proteins zu dem so gebildeten Gemisch, um an 1a- oder 24R,25-Dihydroxy-Vitamin-D3 und an das lod-Radioisotop-markierte 1a- oder 24R,25-Dihydroxy-Vitamin-D3-Derivat kompetitiv zu binden,
die Antrennung des so hergestellten lod-Radioisotop-markiertes 1a- oder 24R,25-Dihydroxy-Vitamin-D3-Derivat-anti-1a-oder -24R,25-Dihydroxy-Vitamin-D3-Antikörperkomplexes oder des lod-Radioisotop-markiertes 1a- oder 24R,25-Dihydroxy-Vitamin-D3-Derivat-DBP-Komplexes von dem lod-Radioisotop-markierten 1a- oder 24R,25-Dihydroxy-Vitamin-D3-Derivat, und
die Messung der Menge der lod-Radioisotopenmarkierung in gebundener oder freier Form.

8. Verfahren zur Herstellung eines nach einem der Ansprüche 4 bis 6 definierten lod-Radioisotop-markierten 1a-oder 24R,25-Dihydroxy-Vitamin-D3-Derivats, worin ein nach Anspruch 1 definiertes 1a-oder 24R,25-Dihydroxy-Vitamin-D3-Derivat mit einer Verbindung der Formel (VII):
Rs-X
wobei R₅ in Anspruch 4 definiert ist und X eine Succinimidyl-N-oxy-, Phthalimidyl-N-oxy-, 5-Norbornen-2,3-dicarboximidyl-N-oxy- oder Maleimidyl-N-oxy-Gruppe bedeutet, umgesetzt wird.

## Revendications

1. Dérivé acide aminé de la 1a ou de la 24R,25-dihydroxy-vitamine D₃, de formule (I) : 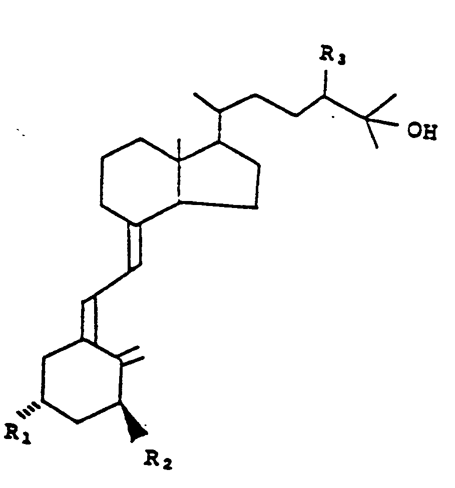 dans laquelle :
R₁ est OH ou -O-CO-A-NH₂ ;
R₂ est H, OH ou -O-CO-A-NH₂; et
R₃ est H, OH ou -O-CO-A-NH₂;
où A est un alkylène en Ci -₁ o, du moment que :
(1) l'un des radicaux R₂ et R₃ est H et l'autre est OH ou -O-CO-A-NH₂;
(2) l'un des radicaux R₁ et R₂ est OH, et l'autre est -O-CO-A-NH₂; ou
(3) l'un des radicaux R₁ et R₃ est OH et l'autre est -0-CO-A-NH₂.

2. Procédé pour produire le dérivé acide aminé de la vitamine D₃ de formule (I) selon la revendication 1, qui consiste à éliminer, en présence d'une base et dans un solvant inerte, un groupe amino-protecteur, à partir d'un dérivé de vitamine D₃ de formule (II) : 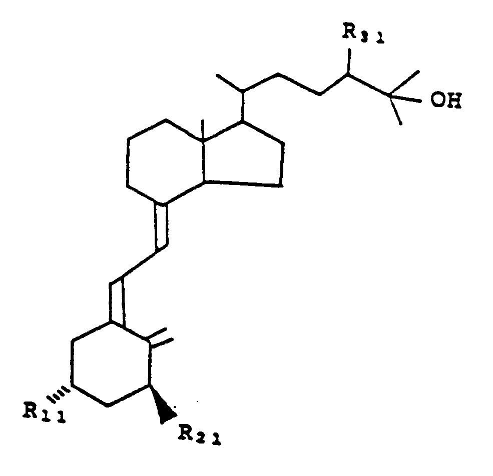 dans laquelle :
R₁₁ est OH ou -O-CO-A-NH-R₄;
R₂₁ est H, OH ou -O-CO-A-NH-R₄; et
R₃₁ est H, OH ou -O-CO-A-NH-R₄;
où A est un alkylène en Ci -₁₀ et R₄ est un groupe amino-protecteur, du moment que :
(1) l'un des radicaux R₂₁ et R₃₁ est H, et l'autre est OH ou -O-CO-A-NH-R₄;
(2) l'un des radicaux R₁₁ et R₂₁ est OH, et l'autre est -0-CO-A-NH-R₄ ; ou
(3) l'un des radicaux R₁₁ et R₃₁ est OH et l'autre est -0-CO-A-NH-R₄.

3. Procédé selon la revendication 2, dans lequel R₄ est le radical 9-fluorénylméthyloxycarbonyle.

4. Dérivé marqué par un radioisotope de l'iode de la 1a ou 24 R, 25-dihydroxy-vitamine D₃ de formule (III) 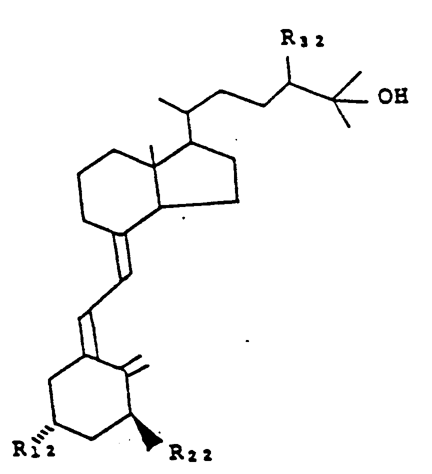 dans laquelle :
R₁₂ est OH ou -O-CO-A-NH-R₅;
R₂₂ est H, OH ou -O-CO-A-NH-R₅; et
R₃₂ est H, OH ou -O-CO-A-NH-R₅;
où A est un alkylène en C₁₋₁₀ et R₅ est un groupe contenant un radioisotope de l'iode, du moment que :
(1) l'un des radicaux R₂₂ et R₃₂ est H, et l'autre est OH ou -O-CO-A-NH-R₅;
(2) l'un des radicaux R₁₂ et R₂₂ est OH, et l'autre est -0-CO-A-NH-R₅ ; ou
(3) l'un des radicaux R₁₂ et R₃₂ est OH et l'autre est -0-CO-A-NH-R₅.

5. Dérivé de la vitamine D₃ selon la revendication 4, dans lequel le radioisotope de l'iode est le ¹²⁵I.

6. Dérivé de la vitamine D₃ selon la revendication 5, dans lequel le groupe contenant un radioisotope de l'iode est le radical 3-(4-hydroxy-3-iodo(¹²⁵I)-phényl)-propionyle ou 3-(3,5-düodo(¹²⁵I)-4-hydroxyphényl)-propionyle.

7. Méthode de dosage de la 1a ou 24R,25-dihydroxy-vitamine D₃ dans un échantillon, qui consiste :
à ajouter, à un échantillon contenant la 1a ou 24R,25-dihydroxy-vitamine D₃ à doser, un dérivé marqué par un radioisotope de l'iode de la 1a ou 24,25-dihydroxy-vitamine D₃, selon l'une quelconque des revendications 4 à 6 ;
à ajouter au mélange ainsi formé des anticorps anti-1a ou 24R,25-dihydroxy-vitamine D₃ ou une protéine fixant la vitamine D, pour fixation compétitive à la 1a ou 24,25-dihydroxy-vitamine D₃ et au dérivé marqué par un radioisotope de l'iode de la 1α ou 24,25-dihydroxy-vitamine D₃ ;
à séparer, d'avec le dérivé marqué par un radioisotope de l'iode de la 1a ou 24R,25-dihydroxy-vitamine D₃, le complexe, ainsi produit, du dérivé marqué par un radioisotope de l'iode de la 1a ou 24R,25-dihydroxy-vitamine D₃ et de l'anticorps anti-1a ou 24R,25-dihydroxy-vitamine D₃, ou encore le complexe constitué d'un dérivé marqué par un radioisotope de l'iode de la 1a ou 24R,25-dihydroxy-vitamine D₃ et de DBP ; et
à mesurer la quantité du marqueur au radioisotope de l'iode sous sa forme liée ou libre.

8. Procédé pour préparer un dérivé marqué par un radioisotope de l'iode de la 1a ou 24,25-dihydroxy-vitamine D₃, selon l'une quelconque des revendications 4 à 6, dans lequel on fait réagir un dérivé de 1α ou 24,25-dihydroxy-vitamine D₃ selon la revendication 1, avec un composé de formule (VII) :
Rs-X
dans laquelle R₅ est tel que défini dans la revendication 4 et X est un groupe succinimidyl-N-oxy, phtalimidyl-N-oxy, 5-norbornène-2,3-dicarboximidyl-N-oxy ou maléimidyl-N-oxy.
